(19) Europäisches Patentamt
European Patent Office
Office européen des brevets

(11) Publication number: **0 111 951 B1**

(12) **EUROPEAN PATENT SPECIFICATION**

(45) Date of publication of patent specification: **26.03.86**

(51) Int. Cl.$^4$: **C 07 C 1/04** // B01J29/28

(21) Application number: **83201659.6**

(22) Date of filing: **22.11.83**

(54) **Process for the preparation of hydrocarbons.**

(30) Priority: **17.12.82 NL 8204884**

(43) Date of publication of application:
**27.06.84 Bulletin 84/26**

(45) Publication of the grant of the patent:
**26.03.86 Bulletin 86/13**

(84) Designated Contracting States:
**BE DE FR GB IT NL**

(56) References cited:
**EP-A-0 018 683**
**EP-A-0 039 964**
**EP-A-0 051 326**
**US-A-3 702 886**
**US-A-4 086 262**
**US-A-4 188 336**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Post, Martin Franciscus Maria**
**Badhuisweg 3**
**NL-1031 CM Amsterdam (NL)**

(74) Representative: **Aalbers, Onno et al**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 111 951 B1

**Description**

The invention relates to a process for the preparation of a hydrocarbon mixture by contacting a mixture of carbon monoxide and hydrogen with a mixture of two catalysts, one of which is capable of catalysing the conversion of a $H_2/CO$ mixture into oxygen-containing organic compounds, and the other is a crystalline aluminium silicate which after one hour's calcination in air at 500°C has the following properties:

a) an X-ray powder diffraction pattern in which the strongest lines are the four lines mentioned in Table A

TABLE A

| d(Å) |
|---|
| 11.1 ±0.2 |
| 10.0 ±0.2 |
| 3.84±0.07 |
| 3.72±0.06 and |

b) in the formula which represents the composition of the silicate expressed in moles of the oxides the $SiO_2/Al_2O_3$ molar ratio is higher than 10,

which silicate has been prepared by maintaining an aqueous mixture comprising one or more compounds of an alkali metal (M), one or more aluminium compounds, one or more silicon compounds which after drying at 120°C and calcination at 500°C yield a product having an $SiO_2$ content of more than 90 %w, and one or more quaternaryalkylammonium compounds ($R_4N$), in which mixture the following compounds are present in the following molar ratio expressed—with the exception of the quaternary alkylammonium compounds—in moles of the oxides

$M_2O:SiO_2$=0.01—0.35,
$R_4N:SiO_2$=0.02—0.80
$SiO_2:Al_2O_3$>10, and
$H_2O:SiO_2$=5—100,

at an elevated temperature until the crystalline silicate has formed followed by separating the latter from the mother-liquor and calcination.

Said process is known from EP—A—51326, wherein a further restriction of the $SiO_2/Al_2O_3$ molar ratio in the formula and in the aqueous mixture to 200—750 and 150—1500 respectively has been proposed and wherein as quaternary alkylammonium compound tetrapropylammonium hydroxide has been used, the $[(CH_3H_7)_4N]_2O:SiO_2$ molar ratio in the aqueous mixture being 0.01—0.4.

Surprisingly, it has been found that a crystalline aluminium silicate, having a $SiO_2/Al_2O_3$ molar ratio of between 100 and 250 and which has been prepared by using one or more quaternary alkylammonium halides ($R_4NX$) and by applying a $SiO_2:Al_2O_3$ molar ratio and $R_4NX:SiO_2$ molar ratio in the aqueous mixture of 100—350 and 0.02—0.40 respectively, shows an improved stability when used in the above process compared with those silicates which have been prepared by using tetrapropylammonium hydroxide.

Therefore, the present invention is concerned with a process for the preparation of a hydrocarbon mixture by contacting a mixture of carbon monoxide and hydrogen with a mixture of two catalysts, one of which is capable of catalysing the conversion of a $H_2/CO$ mixture into oxygen-containing organic compounds, and the other is a crystalline aluminium silicate which after one hour's calcination in air at 500°C has the following properties:

a) an X-ray powder diffraction pattern in which the strongest lines are the four lines mentioned in Table A

TABLE A

| d(Å) |
|---|
| 11.1 ±0.2 |
| 10.0 ±0.2 |
| 3.84±0.07 |
| 3.72±0.06 and |

b) in the formula which represents the composition of the silicate expressed in moles of the oxides the $SiO_2/Al_2O_3$ molar ratio is higher than 10,

which silicate has been prepared by maintaining an aqueous mixture comprising one or more compounds

of an alkali metal (M), one or more aluminium compounds, one or more silicon compounds which after drying at 120°C and calcination at 500°C yield a product having an $SiO_2$ content of more than 90 %w, and one or more quaternary alkylammonium compounds ($R_4N$), in which mixture the following compounds are present in the following molar ratio expressed—with the exception of the quaternary alkylammonium compounds—in moles of the oxides

$M_2O:SiO_2=0.01—0.35$,
$R_4N:SiO_2=0.02—0.80$,
$SiO_2:Al_2O_3>10$, and
$H_2O:SiO_2=5—100$,

at an elevated temperature until the crystalline silicate has formed followed by separating the latter from the mother-liquor and calcination, characterized in that, the $SiO_2/Al_2O_3$ molar ratio in the formula is between 100 and 250, that the $SiO_2/Al_2O_3$ molar ratio in the aqueous mixture is 100—350, that as quaternary alkylammonium compounds quaternary alkylammonium halides ($R_4NX$), wherein the alkyl groups contain 2—4 carbon atoms, are applied, the molar ratio of $R_4NX:SiO_2$ in the aqueous mixture being 0.02—0.40.

The silicon compounds used in the preparation of the crystalline silicates are silicon compounds which after drying at 120°C and calcination at 500°C yield a product having a $SiO_2$ content of more than 90 %w. For the sake of brevity these silicon compounds will hereinafter be referred to as "silicon compounds having a high $SiO_2$ content". Silicon compounds which yield after drying at 120°C and calcining at 500°C a product having a $SiO_2$ content of at most 90 %w, will be referred to as "silicon compounds having a low $SiO_2$ content" hereinafter. Examples of silicon compounds having a high $SiO_2$ content are amorphous solid silicas, silica sols, silica gels and kieselguhr. An example of a silicon compound having a low $SiO_2$ content is water glass. The preparation of the crystalline silicates is carried out by maintaining the aqueous mixture at an elevated temperature until the silicate has formed and subsequently separating the silicate crystals from the mother-liquor and washing, drying and calcining the crystals.

With the use of $R_4NX$-type compounds there exists a relation between the $R_4N^+/SiO_2$ molar ratio in the aqueous base mixture from which the silicate is prepared and the stability of the catalyst mixture composed by using the silicate. With $R_4NX$-type compounds it was seen that, as higher $R_4NX/SiO_2$ molar ratios were used, the stability of the catalyst mixtures increased at first and then, after a maximum value had been reached, decreased. The $R_4NX/SiO_2$ molar ratio used in the aqueous mixture should lie between 0.02 and 0.40.

In the preparation of the silicates by using an $R_4NX$-type compound the aqueous mixture from which they are prepared must include one or more compounds of an alkali metal (M), notably in a concentration expressed as $M_2O/SiO_2$ molar ratio lying between 0.01 and 0.35. For the preparation of the silicates by using an $R_4NX$-type compound only silicon compounds with a high $SiO_2$ content are eligible.

The stability of the aluminium silicates obtained with $R_4NX$ in the aqueous mixture, was found to be dependent on the $SiO_2/Al_2O_3$ molar ratio. Optimal stabilities were obtained in silicates having a $SiO_2/Al_2O_3$ molar ratio between 100 and 250. Such silicates are prepared when in the aqueous base mixture silicon and aluminium compounds are present in such quantities that their molar ratio, expressed in moles of the oxides is $SiO_2:Al_2O_3=100—350$.

In the process according to the invention the starting material is a $H_2/CO$ mixture. Such a mixture can very suitably be prepared by gasifying heavy carbonaceous materials, such as coal, or by catalytic steam reforming of light hydrocarbons, such as methane.

The process according to the invention is preferably carried out at a temperature of 200—500°C and in particular of 300—450°C, a pressure of 1—150 bar and in particular of 5—100 bar and a space velocity of 50—5000 and in particular 300—3000 Nl gas per litre catalyst per hour.

In the process according to the invention a mixture of two catalysts is used, one of which is capable of catalysing the conversion of a $H_2/CO$ mixture into oxygen-containing organic compounds. Preference is given to the use of catalysts capable of converting a $H_2/CO$ mixture into substantially methanol and/or dimethylether. Very suitable compositions are zinc-containing compositions which, in addition to zinc, contain one or more of the metals chromium, copper and aluminium. Examples of suitable metal combinations are zinc-chromium, zinc-chromium-copper and zinc-aluminium-copper. Preference is given to the use of catalysts which, in addition to zinc, contain chromium, in particular catalysts in which the atomic percentage of zinc calculated on the sum of zinc and chromium is at least 60% and in particular 60—80%. The above-mentioned zinc-containing compositions are generally prepared by calcination of one or more precipitates obtained by adding a basic reacting substance to one or more aqueous solutions containing salts of the metals involved. From the zinc-containing compositions thus prepared the present catalyst mixtures can be obtained by mechanically mixing particles of the zinc containing compositions with the crystalline silicate. Another very attractive method of preparing these catalyst mixtures is spray-drying. Spray-drying is a method which has been applied on a commercial scale for many years for preparing small globular particles starting from a solid material or a mixture of solids. The process is carried out by atomizing a dispersion in water of the material to be spray-dried through a nozzle or from a rotating disc into hot gas. The process is excellently suited to effect a very intimate contact between different materials. The preparation of the present catalyst mixtures by spray-drying is carried out by dispersing the crystalline silicate in water together with one or more precipitates in which zinc and one or more metals chosen from chromium, copper and aluminium are present and which precipitates have been

obtained by adding a basic reacting substance to one or more aqueous solutions of salts of the metals involved and using the dispersion thus obtained to prepare the desired catalyst mixture by spray-drying. In view of form, size and strength of the catalyst particles prepared by spray-drying they are very suitable for use in a fluidized state.

As regards the ratio of the two catalysts in the catalyst mixtures used in the process according to the invention per part by weight of the crystalline aluminium silicate catalyst these mixtures preferably contain 2.5—12.5 pbw of the catalyst having activity for the conversion of a $H_2/CO$ mixture.

The crystalline aluminium silicates used as catalyst components in the process according to the invention are defined among other things with the aid of the X-ray powder diffraction pattern which they display after one hour's calcination in air at 500°C. In this pattern the strongest lines should be the four lines mentioned in Table A. The complete X-ray powder diffraction pattern of a typical example of the present crystalline aluminium silicates after one hour's calcination in air at 500°C is given in Table B.

TABLE B

| d(Å) | Rel. int. | d(Å) | Rel. int. |
|---|---|---|---|
| 11.1 | 100 | 3.84 (D) | 57 |
| 10.0 (D) | 70 | 3.72 (D) | 31 |
| 8.93 | 1 | 3.63 | 16 |
| 7.99 | 1 | 3.47 | <1 |
| 7.42 | 2 | 3.43 | 5 |
| 6.68 | 7 | 3.34 | 2 |
| 6.35 | 11 | 3.30 | 5 |
| 5.97 | 17 | 3.25 | 1 |
| 5.70 | 7 | 3.05 | 8 |
| 5.56 | 10 | 2.98 | 11 |
| 5.35 | 2 | 2.96 | 3 |
| 4.98 (D) | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |
| 4.00 | 4 | | |

(D)=doublet

In the process according to the invention a catalyst mixture is used the silicate component of which has been prepared by using a quaternary alkylammonium halide $R_4NX$ whose alkyl groups contain 2—4 carbon atoms. In the preparation of the silicate preference is given to the use of a compound $R_4NX$ wherein the alkyl groups are similar and contain three or four carbon atoms. The compound $R_4NX$ is preferably used in the form of a bromide. In the aqueous base mixture from which the silicate component is prepared the $R_4NX/SiO_2$ molar ratio is 0.02—0.40. The crystalline aluminium silicates used in the catalyst mixtures have a $SiO_2/Al_2O_3$ molar ratio between 100 and 250. In the preparation of the crystalline silicates the starting material may very suitably be a base mixture in which the alkali metal compound present is a sodium compound and the silicon compound amorphous silica.

The silicates prepared as described hereinbefore contain alkali metal ions. By using appropriate exchange methods those can be replaced by other cations, such as hydrogen ions or ammonium ions. The crystalline aluminium silicates used in the present catalyst mixtures preferably have an alkali metal content lower than 0.05%w.

The process according to the invention in which a hydrocarbon mixture is prepared from a $H_2/CO$ mixture can very suitably be carried out as an independent process in which the conversion of the $H_2/CO$ mixture is effected in a single step. Unconverted synthesis gas may optionally be recirculated. The process according to the invention may also be used very suitably as part of a multi-step process for the conversion of $H_2/CO$ mixtures into hydrocarbon mixtures. In that case two possibilities offer, viz.

a) the process is carried out as the first step of a two-step process in which $H_2$ and CO present in the reaction product of the first step are contacted in a second step, together with other components from that reaction product if desired, with other components from that reaction product if desired, with a catalyst containing one or more metal components with activity for the conversion of $H_2/CO$ mixture into paraffinic hydrocarbons, which metal components have been chosen from the group formed by cobalt, nickel and ruthenium.

b) The process is carried out as the first step of a three-step process in which the first two steps are carried out as described under a) and in which in the second step the catalyst used is a zirconium, titanium or chromium-promoted cobalt catalyst supported on silica as the carrier, which catalyst has been prepared by impregnation and/or kneading. In this three-step process advantage is taken of the fact that the high-boiling part of the reaction product of the second step can be converted in a high yield into middle distillates by using a catalytic hydrotreatment.

The three-step process mentioned under b) involves carrying out an additional catalytic hydrotreatment as the third step, following the two-step process as mentioned under a). As the feed for the catalytic hydrotreatment at least the part of the reaction product of the second step is chosen whose initial boiling point lies above the final boiling point of the heaviest middle distillate desired as final product. The hydrotreatment, which is characterized by a very low hydrogen consumption, yields middle distillates of a considerably lower pour point than those obtained in the direct conversion of a $H_2/CO$ mixture according to Fischer-Tropsch. Very suitable catalysts for carrying out the catalytic hydrotreatment are those containing one or more noble metals from Group VIII supported on a carrier.

The invention is now illustrated with the aid of the following example.

Example

15 Crystalline aluminium silicates (silicates 1—15) were prepared from mixtures of NaOH, amorphous silica, $NaAlO_2$ and $(C_3H_7)_4NOH$ or $(C_3H_7)_4NBr$ in water. In addition one crystalline aluminium silicate (silicate 16) was prepared from a mixture of NaOH, amorphous silica, $NaAlO_2$ $(C_3H_7)_3N$ and $C_3H_7Br$ in water. Finally one crystalline aluminium silicate (silicate 17) was prepared from a mixture of water glass, $Al_2(SO_4)_3$, $H_2SO_4$ and $(C_3H_7)_4NBr$ in water. The preparation of the silicates was carried out by maintaining the mixtures in an autoclave under autogenous pressure with stirring at 150°C for 24 hours. After cooling of the reaction mixtures the silicates were filtered off, washed with water until the pH of the wash water was about 8, dried at 120°C and calcined in air during one hour at 500°C. Silicates 1—17 had the following properties:

a) an X-ray powder diffraction pattern substantially corresponding to that mentioned in Table B, and

b) values for the $SiO_2/Al_2O_3$ molar ratios as listed in Table C.

TABLE C

| Silicate No. | $SiO_2/Al_2O_3$ molar ratio |
|---|---|
| 1 | 490 |
| 2 | 515 |
| 3 | 196 |
| 4 | 203 |
| 5 | 195 |
| 6 | 180 |
| 7 | 221 |
| 8 | 183 |
| 9 | 171 |
| 10 | 170 |
| 11 | 167 |
| 12 | 495 |
| 13 | 405 |
| 14 | 59 |
| 15 | 73 |
| 16 | 164 |
| 17 | 173 |

The amorphous silica used in the preparation of silicates 1—16 after drying at 120°C and calcination at 500°C yielded a product 99.97%w of which consisted of $SiO_2$. The water glass used in the preparation of silicate 17 after drying at 120°C and calcination at 500°C yielded a product 78%w of which consisted of $SiO_2$.

The molar composition of the aqueous mixtures used to prepare silicates 1—15 may be rendered as follows:

$$w\ Na_2O \cdot x\ (C_3H_7)_4NOH \cdot y\ (C_3H_7)_4NBr \cdot z\ Al_2O_3 \cdot 25\ SiO_2 \cdot 450\ H_2O$$

where w, x, y and z have the values given in Table D.

TABLE D

| Silicate No. | w | x | y | z |
|---|---|---|---|---|
| 1 | 1 | 3 | — | 0.044 |
| 2 | 1 | 9 | — | 0.037 |
| 3 | 1 | 1 | — | 0.125 |
| 4 | 1 | 3 | — | 0.125 |
| 5 | 1 | 9 | — | 0.095 |
| 6 | 1 | 15 | — | 0.071 |
| 7 | 3 | 3 | — | 0.075 |
| 8 | 3 | 9 | — | 0.058 |
| 9 | 4 | — | 15 | 0.125 |
| 10 | 4 | — | 9 | 0.125 |
| 11 | 4 | — | 3 | 0.125 |
| 12 | 4 | — | 9 | 0.042 |
| 13 | 2 | — | 1 | 0.063 |
| 14 | 4 | — | 3 | 0.333 |
| 15 | 2 | — | 7 | 0.333 |

The molar compositions of the aqueous mixtures from which silicates 16 and 17 are prepared may be rendered as follows:

For silicate 16

$$4\ Na_2O \cdot 9\ (C_3H_7)_3N \cdot 9\ C_3H_7Br \cdot 0.125\ Al_2O_3 \cdot 25\ SiO_2 \cdot 450\ H_2O$$

For silicate 17

$$1.2\ Na_2O \cdot 6.0\ Na_2SO_4 \cdot 2.5\ (C_3H_7)_4NBr \cdot 0.14\ Al_2O_3 \cdot 25\ SiO_2 \cdot 1000\ H_2O.$$

Silicates 1—17 were used to prepare silicates 18—34, respectively, by boiling silicates 1—17 with a 1.0 molar $NH_4NO_3$ solution, washing with water, boiling again with a 1.0 molar $NH_4NO_3$ solution and washing, drying at 120°C and calcination at 500°C. Subsequently 17 catalyst mixtures (catalyst mixtures A—Q) were prepared by mixing a $ZnO—Cr_2O_3$ composition with each one of silicates 18—34. The atomic Zn percentage of the $ZnO—Cr_2O_3$ composition, based on the sum of Zn and Cr, was 70%. Catalyst mixtures A—Q contained 10 pbw of the $ZnO—Cr_2O_3$ composition per pbw silicate.

Catalyst mixtures A—Q were tested for the production of a hydrocarbon mixture from a $H_2/CO$ mixture. Catalysts A—Q were tested in a 50 ml reactor containing a fixed catalyst bed of 10 g in weight. In 17 experiments (Experiments 1—17) a $H_2/CO$ mixture having a $H_2/CO$ molar ratio of 0.5 was passed over each one of catalyst mixtures A—Q at a temperature of 375°C, a pressure of 60 bar and a space velocity of 850 $Nl \cdot kg^{-1} \cdot h^{-1}$. The results of these experiments are listed in Table E.

TABLE E

| Exp. No. | Catalyst mixture No. | Silicate No. | Conversion of synthesis gas after 10 hours %v | Stability expressed as difference between conversion after 10 and 200 hours | $C_3^+$ selectivity averaged over 200 hours, %w | $C_5^+$ selectivity averaged over 200 hours, %w |
|---|---|---|---|---|---|---|
| 1 | A | 18 | 52 | 9 | 93 | 75 |
| 2 | B | 19 | 54 | 4 | 93 | 74 |
| 3 | C | 20 | 60 | 13 | 93 | 72 |
| 4 | D | 21 | 58 | 10 | 94 | 71 |
| 5 | E | 22 | 61 | 4 | 94 | 72 |
| 6 | F | 23 | 62 | 3 | 94 | 70 |
| 7 | G | 24 | 62 | 7 | 85 | 60 |
| 8 | H | 25 | 63 | 6 | 93 | 71 |
| 9 | I | 26 | 66 | 10 | 95 | 72 |
| 10 | J | 27 | 65 | <1 | 94 | 74 |
| 11 | K | 28 | 64 | <1 | 94 | 73 |
| 12 | L | 29 | 62 | 2 | 96 | 80 |
| 13 | M | 30 | 61 | 3 | 97 | 78 |
| 14 | N | 31 | 71 | 4 | 93 | 69 |
| 15 | O | 32 | 68 | 4 | 94 | 71 |
| 16 | P | 33 | 58 | 8 | 93 | 71 |
| 17 | Q | 34 | 64 | 8 | 93 | 72 |

Of Experiments 1—17 given in Table E only Experiments 10 and 11 are experiments according to the invention. Experiments 1—9 and 12—17 fall outside the scope of the invention. They have been included in the patent application for comparison. In Experiments 1—8 catalyst mixtures were used which contained silicates prepared by using a tetra alkylammonium hydroxide ($R_4NOH$) compound. In Experiment 9 the catalyst mixture used contained a silicate which indeed had been prepared by using an $R_4NX$-type compound, but in a quantity corresponding to an $R_4NX/SiO_2$ molar ratio higher than 0.40. In Experiment 16 a catalyst mixture was used which contained a silicate prepared by using a mixture of an $R_3N$-type compound with an RX-type compound in a 1:1 molar ratio, instead of an $R_4NX$-type compound. In Experiment 17 a catalyst mixture was used which contained a silicate prepared by using a silicon compound with a low $SiO_2$ content.

In Experiments 12—15 catalyst mixtures containing a silicate prepared by using $R_4NX$ were used, but the $SiO_2/Al_2O_3$ molar ratios of the silicates are beyond the range according to the invention.

As regards the results given in Table E the following may be remarked:

a) Comparison of the results of Experiments 1 and 2, 3—6 and 7 and 8 in relation with each other shows that the stability of the catalyst mixtures increases as higher $R_4NOH/SiO_2$ molar ratios are used in the preparation of the silicate. This holds good both for the silicates prepared by using a $M_2O/SiO_2$ molar ratio of 0.04 (Experiments 1—6) and for silicates prepared by using a $M_2O/SiO_2$ molar ratio of 0.12 (Experiments 7 and 8).

b) Comparison of the results of Experiments 5 and 8 (both carried out by using a catalyst mixture in which the silicates had comparable $SiO_2/Al_2O_3$ molar ratios and had been prepared by using the same high $R_4NOH/SiO_2$ molar ratio) shows that in combination with the high $R_4NOH/SiO_2$ molar ratio used an increase of the $M_2O/SiO_2$ molar ratio leads to a decrease in stability.

c) Comparison of the results of Experiments 10 and 11, 4 and 5 and 7 and 8 (carried out by using catalyst mixtures in which the silicates had comparable $SiO_2/Al_2O_3$ molar ratios) shows that replacing $R_4NOH$ by $R_4NX$ results in very considerable stability enhancement. Furthermore, catalyst mixtures prepared by using $R_4NX$ have higher activities and higher selectivities. Comparison of the results of Experiments 9 and 6 shows that the stability-promoting effect produced by replacing $R_4NOH$ by $R_4NX$ is absent when $R_4NX/SiO_2$ molar ratios higher than 0.40 are used.

d) Comparison of the results of Experiments 10 and 11 with those of Experiments 12—15 shows that crystalline aluminium silicates prepared by using $R_4NX$-type compounds having either a $SiO_2/Al_2O_3$ molar ratio below 100 or a $SiO_2/Al_2O_3$ molar ratio above 250, have a decreased stability.

e) Comparison of the results of Experiments 10 and 17 shows that use of silicates obtained from silicon compounds having a low $SiO_2$ content has a detrimental effect on the stability of the catalyst mixtures.

f) The results of Experiments 10 and 16 show that the stability of a catalyst containing a silicate, is disadvantageously influenced when in the silicate preparation a combination of $R_3N$ and RX is used instead of $R_4NX$.

**Claims**

1. A process for the preparation of a hydrocarbon mixture by contacting a mixture of carbon monoxide and hydrogen with a mixture of two catalysts, one of which is capable of catalysing the conversion of a $H_2/CO$ mixture into oxygen-containing organic compounds, and the other is a crystalline aluminium silicate which after one hour's calcination in air at 500°C has the following properties:

a) an X-ray powder diffraction pattern in which the strongest lines are the four lines mentioned in Table A

TABLE A

| d(Å) |
|---|
| 11.1 ±0.2 |
| 10.0 ±0.2 |
| 3.84±0.07 |
| 3.72±0.06 and |

b) in the formula which represents the composition of the silicate expressed in moles of the oxides the $SiO_2/Al_2O_3$ molar ratio is higher than 10.

which silicate has been prepared by maintaining an aqueous mixture comprising one or more compounds of an alkali metal (M), one or more aluminium compounds, one or more silicon compounds which after drying at 120°C and calcination at 500°C yield a product having an $SiO_2$ content of more than 90%w, and one or more quaternary alkylammonium compounds ($R_4N$), in which mixture the following compounds are present in the following molar ratio expressed—with the exception of the quaternary alkylammonium compounds—in moles of the oxides

$M_2O:SiO_2=0.01$—0.35,
$R_4N:SiO_2=0.02$—0.80,
$SiO_2:Al_2O_3>10$, and
$H_2O:SiO_2=5$—100,

at an elevated temperature until the crystalline silicate has formed followed by separating the latter from the mother-liquor and calcination, characterized in that, the $SiO_2/Al_2O_3$ molar ratio in the formula is between 100 and 250, that the $SiO_2/Al_2O_3$ molar ratio in the aqueous mixture is 100—350, that as quaternary alkylammonium compounds quaternary alkylammonium halides ($R_4NX$), wherein the alkyl groups contain 2—4 carbon atoms, are applied, the molar ratio of $R_4NX:SiO_2$ in the aqueous mixture being 0.02—0.40.

2. A process as claimed in claim 1, characterized in that the catalyst mixture has been prepared by spray-drying.

3. A process as claimed in any one of claims 1—2, characterized in that per pbw of the crystalline silicate catalyst the catalyst mixture contains 2.5—12.5 pbw of the catalyst having activity for the conversion of a $H_2/CO$ mixture.

4. A process as claimed in any one of claims 1—3, characterized in that in the preparation of the crystalline silicate an $R_4NX$ compound is used in which the alkyl groups are similar and contain three or four carbon atoms.

5. A process as claimed in any one of claims 1—4, characterized in that in the preparation of the crystalline silicate the $R_4NX$ compound is used as a bromide.

**Patentansprüche**

1. Ein Verfahren zur Herstellung einer Kohlenwasserstoffmischung durch Inberührungbringen einer Mischung aus Kohlenmonoxid und Wasserstoff mit einer Mischung aus zwei Katalysatoren, von denen der eine in der Lage ist, die Umwandlung einer $H_2$/CO-Mischung in sauerstoffhaltige organische Verbindungen zu katalysieren, und der andere ein kristallines Alumosilikat darstellt, das nach einstündiger Kalzinierung in Luft bei 500°C die nachstehenden Eigenschaften aufweist:

a) ein Röntgenpulverdiagramm, in welchem die stärksten Linien die vier nachstehend in Tabelle A angegebenen Linien sind.

TABELLE A

| d(Å) |
|---|
| 11.1 ±0.2 |
| 10.0 ±0.2 |
| 3.84±0.07 |
| 3.72±0.06 |

b) in der die Zusammensetzung des Silikats wiedergebende Formel, ausgedrückt in Molen der Oxide, das Molverhältnis $SiO_2/Al_2O_3$ einen Wert höher als 10 aufweist,

welches Silikat hergestellt worden ist durch Halten einer wässrigen Mischung auf erhöhter Temperatur, welche eine oder mehrere Verbindungen eines Alkalimetalls (M), eine oder mehrere Aluminiumverbindungen, eine oder mehrere Siliciumverbindungen, welche nach Trocknen bei 120°C und Kalzinieren bei 500°C ein Produkt mit einem $SiO_2$-Gehalt von mehr als 90 Gewichtsprozent liefern, und eine oder mehrere quaternäre Alkylammoniumverbindungen ($R_4N$), enthält, in welcher Mischung die nachstehenden Verbindungen in den nachstehend angegebenen Molverhältnissen, ausgedrückt in Molen der Oxide—mit Ausnahme der quaternären Alkylammoniumverbindungen—vorliegen

$M_2O:SiO_2=0.01$—0.35
$R_4N:SiO_2=0.02$—0.80
$SiO_2:Al_2O_3>10$ und
$H_2O:SiO_2=5$—100,

bis sich das kristalline Silikat gebildet hat, nachfolgendes Abtrennen des letzteren aus der Mutterlauge und Kalzinieren, dadurch gekennzeichnet, daß das $SiO_2/Al_2O_3$-Molverhältnis in der Formel einen Wert zwischen 100 und 250 hat, daß das $SiO_2/Al_2O_3$-Molverhältnis in der wässrigen Mischung einen Wert von 100 bis 350 hat, daß als quaternäre Alkylammoniumverbindungen quaternäre Alkylammoniumhalogenide ($R_4NX$) verwendet werden, bei denen die Alkylgruppe 2 bis 4 Kohlenstoffatome aufweisen, und das Molverhältnis von $R_4NX:SiO_2$ in der wässrigen Mischung einen Wert zwischen 0.02 bis 0.40 aufweist.

2. Ein Verfahren wie in Anspruch 1 beansprucht, dadurch gekennzeichnet, daß die Katalysatormischung durch Sprühtrocknung hergestellt worden ist.

3. Ein Verfahren wie in irgendeinem der Ansprüche 1 bis 2 beansprucht, dadurch gekennzeichnet, daß die Katalysatormischung je Gewichtsteil des als kristallines Silikat vorliegenden Katalysators 2,5 bis 12,5 Gewichtsteile des Katalysators mit Aktivität für die Umwandlung einer $H_2$/CO-Mischung enthält.

4. Ein Verfahren wie in irgendeinem der Ansprüche 1 bis 3 beansprucht, dadurch gekennzeichnet, daß bei der Herstellung des kristallinen Silikats eine $R_4NX$-Verbindung verwendet wird, in welcher die Alkylgruppen gleich sind und 3 oder 4 Kohlenstoffatome enthalten.

5. Ein Verfahren wie in irgendeinem der Ansprüche 1 bis 4 beansprucht, dadurch gekennzeichnet, daß die bei der Herstellung des kristallinen Silikats verwendete $R_4NX$-Verbindung ein Bromid ist.

**Revendications**

1. Procédé pour la préparation d'un mélange d'hydrocarbures en mettant en contact un mélange de monoxyde de carbone et d'hydrogène avec un mélange de deux catalyseurs, dont l'un est capable de catalyser la conversion d'un mélange $H_2/CO$ en composés organiques oxygénés, et l'autre est un silicate d'aluminium cristallin qui possède, après une calcination d'une heure dans l'air à 500°C, les propriétés suivantes:

a) un spectre de diffraction X de poudre dans lequel les raies les plus intenses sont les quatre raies mentionnées dans le tableau A

TABLEAU A

| d(Å) |
|---|
| 11,1 ±0,2 |
| 10,0 ±0,2 |
| 3,84±0,07 |
| 3,72±0,06 et |

b) dans la formule qui représente la composition du silicate exprimée en moles des oxydes, le rapport molaire $SiO_2/Al_2O_3$ est supérieur à 10,

ce silicate ayant été préparé en maintenant un mélange aqueux comprenant un ou plusieurs composés d'un métal alcalin (M), un ou plusieurs composés d'aluminium, un ou plusieurs composés de silicium qui donnent, après un séchage à 120°C et une calcination à 500°C, un produit ayant une teneur en $SiO_2$ supérieure à 90% en poids, et un ou plusieurs composés alkylammonium quaternaire ($R_4N$), les composés suivants étant présents dans ce mélange dans les rapports molaires suivants exprimés—à l'exception des composés alkylammonium quaternaire—en moles des oxydes

$M_2O:SiO_2=0,01—0,35$,
$R_4N:SiO_2=0,02—0,80$,
$SiO_2:Al_2O_3>10$, et
$H_2O:SiO_2=5—100$,

à une température élevée jusqu'à ce que le silicate cristallin se soit formé, puis en séparant ce dernier de la liqueur mère et en le calcinant, caractérisé en ce que le rapport $SiO_2/Al_2O_3$ dans la formule est compris entre 100 et 250, que le rapport molaire $SiO_2/Al_2O_3$ dans le mélange aqueux est de 100—350, que l'on emploie comme composés alkylammonium quaternaire des halogénures d'alkylammonium quaternaire ($R_4NX$), dans lesquels les groupes alkyle contiennent 2—4 atomes de carbone, le rapport molaire de $R_4NX:SiO_2$ dans le mélange aqueux étant de 0,02—0,40.

2. Procédé selon la revendication 1, caractérisé en ce que le mélange catalytique a été préparé par séchage par atomisation.

3. Procédé selon l'une quelconque des revendications 1 ou 2, caractérisé en ce que le mélange catalytique contient, par partie en poids de catalyseur au silicate cristallin, 2,5—12,5 parties en poids de catalyseur ayant une activité dans la conversion d'un mélange $H_2/CO$.

4. Procédé selon l'une quelconque des revendications 1 à 3, caractérisé en ce que, dans la préparation du silicate cristallin, on utilise un composé $R_4NX$ dans lequel les groupes alkyle sont identiques et contiennent trois ou quatre atomes de carbone.

5. Procédé selon l'une quelconque des revendications 1 à 4, caractérisé en ce que, dans la préparation du silicate cristallin, on utilise le composé $R_4NX$ sous forme de bromure.